Europäisches Patentamt

European Patent Office

Office européen des brevets

(19)

(11) Veröffentlichungsnummer: **0 255 797**
**B1**

(12) # EUROPÄISCHE PATENTSCHRIFT

(45) Veröffentlichungstag der Patentschrift:
**13.06.90**

(51) Int. Cl.⁵: **A61F 2/30, A61F 2/02**

(21) Anmeldenummer: **87730089.7**

(22) Anmeldetag: **04.08.87**

(54) **Verfahren und Vorrichtung zur Herstellung einer Endoprothese mit individueller Anpassung.**

(30) Priorität: **06.08.86 DE 3626549**

(43) Veröffentlichungstag der Anmeldung:
**10.02.88 Patentblatt 88/6**

(45) Bekanntmachung des Hinweises auf die Patenterteilung:
**13.06.90 Patentblatt 90/24**

(84) Benannte Vertragsstaaten:
**CH DE ES FR GB IT LI**

(56) Entgegenhaltungen:
**EP-A- 0 093 869**
**EP-A- 0 097 001**
**EP-A- 0 146 491**

(73) Patentinhaber: **MECRON MEDIZINISCHE PRODUKTE GMBH, Nunsdorfer Ring 23-27, D-1000 Berlin 48(DE)**

(72) Erfinder: **Ahrens, Uwe, Nürnberger Strasse 46, D-1000 Berlin 30(DE)**
Erfinder: **Kranz, Curt, Kufsteiner Strasse 12, D-1000 Berlin 62(DE)**

(74) Vertreter: **Christiansen, Henning, Dipl.-Ing., Patentanwalt CHRISTIANSEN Pacelliallee 43/45, D-1000 Berlin 33(DE)**

## Beschreibung

Die Erfindung betrifft ein Verfahren der im Oberbegriff des Anspruchs I angegebenen Art sowie eine Anordnung zur Durchführung des Verfahrens.

Es sind verschiedene Verfahren zur Herstellung von Endoprothesen mit individueller Anpassung bekannt geworden, bei denen die Herstellung der Endoprothese auf Grund eines durch Computer-Tomographie erzeugten Knochenmodells erfolgt.

Entsprechend wird in den Aufsätzen in der "Zeitschrift für Orthopädie", II8 (I80) Seiten 33I bis 336, "Möglichkeiten geometrischer Röntgenuntersuchung des Beckens für den halbseitigen Beckenersatz" von H. Gerngroß, C. Burin, L. Claes und V. Eckhard sowie in "Fortschritte der Medizin", 27. Jahrgang (I979), Nr. I6, Seiten 78I bis 783, von H.-D. Toner und H. Engelbrecht "Ein neues Verfahren zur Herstellung aloplastischer Spezialimplantate für den Beckenteilersatz" auf die computertomografische Erzeugung von Modellen für den Knochenersatz eingegangen.

Bei den bekannten Verfahren besteht der Nachteil, daß bei exakter Erzeugung eines Modells des zu ersetzenden Knochenbereichs durch Coputertomografie der betreffende Defekt eberıfalls dreidimensional reproduziert wird. Problematisch ist auch die Erzeugung von genau passenden Anschlüssen an zu erhaltende Knochenbereiche, wobei der Arzt durch Fehlanpassungen in Übergangsbereichen auf schwerwiegende Probleme hinsichtlich Befestigung und Festigkeit des künftigen Verbundes stößt.

Der im Anspruch I angegebenen Erfindung liegt die Aufgabe zugrunde, ein Verfahren der eingangs genannten Art anzugeben, welches es ermöglicht, mit Mitteln der Computertomografie erzeugte Modelle so herzustellen, wie sie dem Knochen in intaktem Zustand entsprechen. Das Verfahren eignet sich sowohl für Schaftprothesen, die an vorgefundene Knochenbereiche anzupassen sind, als auch für zu ersetzende Knochenteile, die an die verbleibenden Knochenbereiche anzupassen sind.

Besonders vorteilhaft ist dabei, daß durch die jeweilige symmetrische Knochenpartie in der Regel ein Vergleichsmodell zur Verfügung steht, welches durch Spiegelung in den gewünschten Modellbereich überführt werden kann, wobei in der Datenverarbeitung durch eine einfache Richtungsvertauschung der die Oberflächenteile bezeichnenden Vektoren bzw. eine entsprechende Änderung der Adressenzuordnung der die Volumen- bzw. Oberflächenelemente enthaltenden Speicherplätze ein Übergang zu spiegelsymmetrischen grafischen Modelldarstellungen möglich ist.

Steht ein derartiges von der anderen Körperhälfte genommenes Modell nicht zur Verfügung, kann auf "Konfektionsmodelle" zurückgegriffen werden. Dabei läßt sich der defekte Bereich durch entsprechende Knochenbereiche eines Modells mit Maßen intakter Knochen ersetzen, die aufgrund statistischer Durchschnittswertmaße gewonnen wurden und entsprechend unterschiedlichen Körpergrößen und Konstitutionen gespeichert abrufbar sind. Zwischen den unterschiedlichen Größen, die mit den Konfektionsgrößen für Oberbekleidung vergleichbar sind, kann durch Interpolation jede beliebige Zwischengröße erzeugt werden.

Die aufgrund der Verwendung von symmetrischen Knochenbereichen derselben Person und die aufgrund statistischer Durchschnittswerte gewonnenen Vergleichsmodelle werden bevorzugt zur Kontrolle des jeweils auf andere Weise gewonnenen Herstellungsmodells herangezogen.

Insbesondere geeignet ist ein Iterationsverfahren, mit dem die zu ersetzenden Knochenpartien beliebig exakt angepaßt werden können auch wenn nach der jeweiligen symmetrischen Körperpartie kein Modell erzeugt werden kann. Dabei wird zunächst eine Anpassung des statistischen Durchschnittsmodells an den den geschädigten Teil enthaltenden Bereich vorgenommen, wobei anschließend die abweichenden Bereiche, die entweder automatisch oder manuell ermittelt werden, die den geschädigten Bereichen entsprechenden Teile des Modells ersetzen. Mit dem dann erhaltenen (provisorisch ergänzten) Modell des zu behandelnden Knochenbereichs wird ein erneuter Abgleich mit den vorhandenen gespeicherten, aufgrund statistischer Werte gewonnenen Modellen vorgenommen. Hier sind jetzt auch Interpolationen oder örtliche Verzerrungen unter Wahrung der geschlossenen Kontur möglich. Das Ergebnis ist eine verbesserte Anpassung, wobei das nunmehr erhaltenen Vergleichsmodell das erste statistische Durchschnittswertmodell ersetzt und wieder zum Ermitteln der abweichenden Bereiche durch Vergleich mit dem ursprünglichen, von den zu behandelnden Knochenbereichen genommenden Modell herangezogen wird. Auf diese Weise wird eine iterative Anpassung erreicht, wobei die zu ersetzenden Knochenbereiche wegen ihrer krankheitsbedingten Abweichungen selbsttätig von dem Iterationsverfahren ausgenommen werden. Die räumliche Anpassung des konfektionierten Modells erfolgt dabei bevorzugt mit allen Möglichkeiten der Verarbeitung von grafischen Daten wie örtliche Dehnung oder Stauchung, Torsion oder Biegung, wobei diese Variationen im Rahmen des iterativen Verfahrens bevorzugt nacheinander ausgeführt werden unter Beachtung der Differenzvolumina der zu vergleichenden Modelle als Abgleichkriterium.

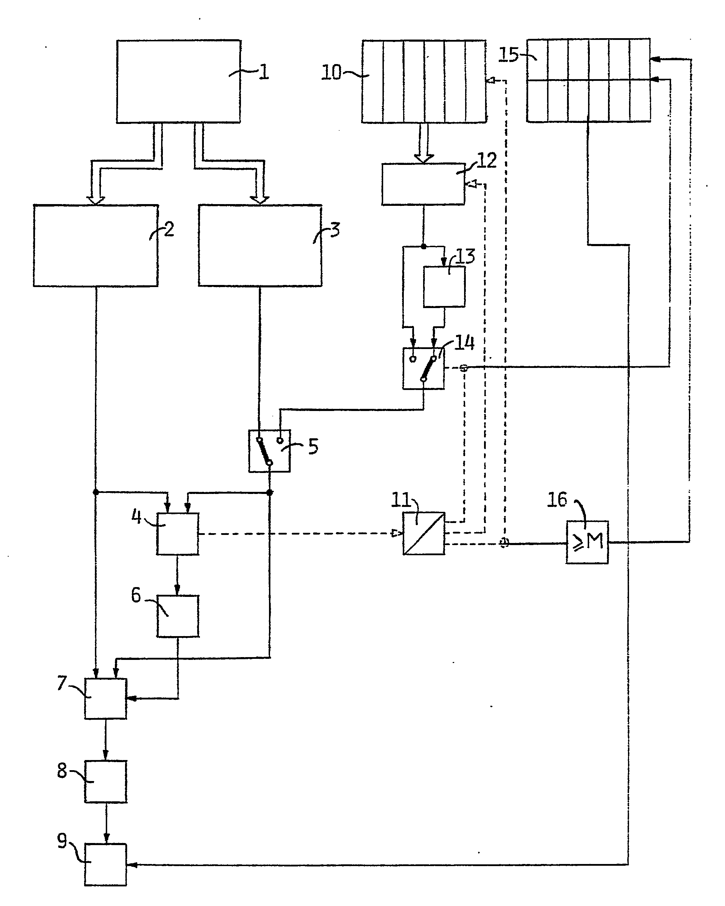

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Die einzige Figur gibt eine blockschaltungsmäßige Repräsentation eines bevorzugten Ausführungsbeispiels wieder.

Block I repräsentiert den Vorgang der computertomografischen Aufnahme der zu ersetzenden Knochenbereiche und des entsprechenden symmetrischen Knochenbereiches desselben Patienten. Die erhaltenen Computer-Tomogramme werden mittels grafischer Verfahren in durch digitale Datenverarbeitung erfaßbare Modelle umgewandelt und in Speichern 2 und 3 festgehalten, wobei der Speicher 2 den zu ersetzenden Bereich und der Speicher 3 den dazu symmetrischen Bereich beinhaltet. Die Inhalte der Speicher 2 und 3 gelangen zu einer Vergleicherstufe 4, wobei in den Weg vom Speicher 3 zur Vergleicherstufe 4 eine Datenweiche 5 eingeschaltet ist. Als Ergebnis des Vergleichs 4 werden in einem weiteren Speicher 6 diejenigen räumlichen Bereiche festgehalten, für die die Abweichung einen vorgegebenen Mindestbetrag überschreitet. Diese räumlichen Bereiche sind datenmäßig entweder gekennzeichnet durch die Adressen, die den betreffenden Modellteilen bei der Datenverarbeitung zugeordnet sind oder entsprechende Vektoren, welche auf diese räumlichen Bereiche zeigen.

Die Stufe 6 steuert eine Einfügungsstufe 7, welche ein Mischen des im Speicher 2 enthaltenen Modells mit den am Ausgang der Datenweiche 5 anstehenden Daten ermöglicht. Das Mischen erfolgt dabei in der Weise, daß die durch die im Speicher 6 gekennzeichneten räumlichen Bereiche enthaltenen Teile des Modells 2 ersetzt werden durch die entsprechenden Abschnitte des Modells, welches mittels der Datenweiche 5 der Einfügungsstufe 7 zugeleitet wird. Bei dem in der Figur dargestellten Fall handelt es sich dabei um das im Speicher 3 enthaltene symmetrische Modell. Nach dem Ersetzen der vom symmetrischen Vergleichsknochenaufbau abweichenden Bereiche erfolgt in einer Stufe 8 eine Anpassung der Übergänge in der Weise, daß sprungartige Übergänge ausgeglichen werden, so daß das Herstellungsmodell einen kontinuierlichen (glatten) Oberflächenverlauf aufweist. Dieses Modell dient als Grundlage für die Stufe der Herstellung 9.

Um ein genau angepaßtes Herstellungsmodell auch dann zu erzielen, wenn ein entsprechender symmetrischer Bereich nicht zur Verfügung steht oder ebenfalls defekt ist bzw. zur Kontrolle des zuvor gewonnenen Modells ist der Ausgang der Datenweiche wahlweise mit in einem weiteren Speicher 10 vorhandenen Modellen abgleichbar, welche in unterschiedlichen Stufen entsprechend den verschiedenen Körpergrößen und Konstitutionen festgehalten sind. In diesem Fall erfolgt ein Vergleich zwischen dem computertomografisch aufgenommenen Modell 2 und dem aufgrund statistischer Erfahrungen aufgenommenen Modellen 10 in dem Vergleicher 4, wobei nach einem Abgleich durch eine Schaltstufe 11 der Speicher 10 in seiner Adressierung so lange verändert wird, bis die relative Abweichung, die insgesamt durch die Vergleichsstufe 4 ermittelt wird, ein Minimum ist. Mittels einer Interpolationsstufe 12 lassen sich durch Interpolation zwischen den im Speicher 10 enthaltenen Größenstufen noch zusätzlich Zwischengrößen (oder auch örtliche Verzerrungen unter Wahrung der kontinuierlichen Raumform) erzielen. Bevorzugt sind im Speicher 10 jeweils nur die Modelle für eine Körperhälfte datenmäßig abgelegt, wobei die Umschaltung auf die jeweils andere symmetrische Hälfte durch eine Spiegelungsstufe 13 erfolgt, welche durch eine entsprechende Änderung der Adressierung oder Wertzuweisung ein für die weitere Verarbeitung spiegelsymmetrisches Modell erzeugt. Die Umschaltung zwischen den beiden symmetrischen Modellteilen erfolgt durch einen Schalter 14.

Wie bei dem Vergleich mit dem symmetrischen Modell wird entsprechend durch die Stufe 6 der durch den im Speicher 10 enthaltenden Modellteil zu ersetzende Bereich ermittelt. Hierbei kann iterativ vorgegangen werden, in der Weise, daß das nunmehr erzeugte Herstellungsmodell, bei dem ein Knochendefekt beseitigt ist, in den Speicher 2 überführt wird und nun durch die Vergleichsstufe 4 eine erneute Anpassung den im Speicher 10 enthaltenden Modellen vorgenommen wird. Das jetzt erhaltene, dem künftigen durch Implantation einer Endoprothese ergänzten Knochenbereich am besten entsprechende Modell wird nunmehr als Grundlage für einen weiteren Vergleich mit dem zugehörigen im Speicher 10 adressierten Modellbereich und dem in den Speicher 2 zurück überführten Mofell des noch defekten Knochenbereichs genommen. Wenn jetzt erneut der zu ersetzende Bereich bestimmt wird, so entspricht das daraufhin erhaltene im Speicher enthaltene Herstellungsmodell noch mehr der idealisierten Form. Dieser Abgleich läßt sich iterativ mehrfach wiederholen. Abschließend wer den noch die Grenzflächen von verbleibenden Knochenbereichen und einzufügenden Knochenersatzbereichen an den vorgesehenen Grenz- oder Trennflächen exakt an das zuerst erzeugt Modell des zu behandelnden Knochenbereichs.

Es ist günstig, wenn die Rohlinge, von denen bei der Herstellung des Knochenersatzes ausgegangen wird, in unterschiedlichen Größen vorrätig gehalten werden, so daß der Arbeitsschritt der individuellen Anpassung mit geringstem Bearbeitungsaufwand schnell ausgeführt werden kann.

Bevorzugt werden dabei die Rohlinge in den Größen vorrätig gehalten, wie sie den im Speicher 10 enthaltenen Modellgrößen entsprechen, wobei die Bearbeitung der Rohlinge jeweils so weit fortgeschritten ist, daß sich nur das der zugeordneten Größe und die jeweils kleineren Modellgrößen in den Rohling einbeschreiben lassen.

Auf diese Weise wird dem Herstellungsvorgang automatisch derjenige Rohling zugewiesen, welcher der verwendeten Modellgröße entspricht bzw. bei der Interpolation beteiligten größeren Modell zugeordnet ist.

Die Rohlinge sind dabei gespeichert in einem Behältnis 15. Wobei Größe und Symmetrieform durch dieselben Ausgangssignale des Blocks 11 bestimmt werden, welche einerseits die Auswahl der Modellgröße im Speicher 10 festlegen und andererseits die Schaltweiche 14 ansteuern, welche die Auswahl zwischen der Grundform und der spiegelsymmetrischer Form des betreffenden Modells bestimmt. Die für die Auswahl des geeigneten Rohlings (genau passende bzw. nächst größere Stufe) notwendigen Entscheidungsoperationen werden in der Stufe 16 vorgenommen.

Es ist ersichtlich, daß sich auf diese Weise mittels numerisch gesteuerter Werkzeugmaschinen, insbesondere Fräsmaschinen, individuell angepaßte Knochenersatzteile in kürzester Frist automatisiert auch dann erzeugen lassen, wenn die zu ersetzenden Knochenbereiche auf Grund von Tumoren oder Traumatisierung Defekte aufweisen.

Die Erfindung beschränkt sich in ihrer Ausführung nicht auf die Realisierung mit diskreten logischen Baugruppen, sondern läßt sich vorteilhaft auch mit programmierter Logik - vorzugsweise unter Verwendung von PC-Rechnern - realisieren.

## Patentansprüche

l. Verfahren zur Herstellung einer Endoprothese mit individueller Anpassung, unter Heranziehung eines mittels Computertomographie erzeugten, mindestens in datenmäßiger Repräsentation vorhandenen, dreidimensionalen Modells,
**gekennzeichnet durch**
die Schritte:

al. Erzeugung eines Vergleichsmodells für die entsprechenden Knochenbereiche der anderen - symmetrischen - Körperhälfte der Person und Spiegelung
oder

a2. Erzeugung eines Vergleichsmodells für die entsprechenden Knochenbereiche nach einem der Körpergröße und der übrigen Konstitution angepaßten, nach statistischen Durchschnittswerten aufgenommenen statistischen Durchschnittsmodell des jeweiligen Knochenbereichs,
sowie

b. Diskrimination der Oberflächen von Modell und Vergleichsmodell durch Vergleich in entsprechender räumlicher Ausrichtung,

c. Ermittlung mindestens eines Bereiches, in dem Abweichungen von dem Vergleichsmodell vorhanden sind, welche einen vorgegebenen Längen- oder Volumenbetrag überschreiten, d. Ersetzen der abweichenden Bereiche durch die entsprechenden Bereiche des Vergleichsmodells sowie

e. Herstellung der Endoprothese nach dem so erhaltenen Modell.

2. Verfahren nach Anspruch I, **gekennzeichnet durch**

a2l. die Erzeugung des Vergleichsmodells durch Interpolation zwischen den gespeicherten statistischen Durchschnittsmodellen.

3. Verfahren nach Anspruch 2, **gekennzeichnet durch**

a22. die Erzeugung des Vergleichsmodells durch Größenvariation oder Interpolation unter Abgleich des statistischen Durchschnittsmodells mit den entsprechenden Knochenbereichen der anderen Körperhälfte mit Spiegelung.

4. Verfahren nach Anspruch I oder 2, insbesondere, wenn der entsprechende Knochenbereich der gegenüberliegenden Körperhälfte nicht ungeschädigt zur Verfügung steht,
**gekennzeichnet durch**

a23. die Erzeugung des Vergleichsmodells durch Größenvariation oder Interpolation des statistischen Durchschnittsmodells unter Abgleich mit den entsprechenden Knochenbereichen der zu behandelnden Körperhälfte, wobei nach Schritt d. eine erneute Anpassung gemäß Schritt al. unter Außerachtlassung der gemäß c. abweichenden und gemäß d. ersetzten Bereiche des Modells iterativ erfolgt.

5. Verfahren nach einem der vorangehenden Ansprüche, **gekennzeichnet durch**

dl. die exakte Anpassung der Querschnitte der Endoprothese an die angrenzenden Knochenbereiche.

6. Verfahren nach einem der vorangehenden Ansprüche, **gekennzeichnet durch**

el. die Herstellung der Endoprothese aus dem volumenmäßig kleinsten von mehreren Rohlingen, die jeweils einem von verschiedenen in vorgegebener Stufung nach den unterschiedlichen Körpergrößen erzeugten Modellen einbeschreibbar sind, wobei die von einem zentralen Punkt des Modells am weitesten entfernten Oberflächenteile die Rohaußenflächen des Rohlings höchstens tangieren, in welchen das gemäß den Schritten a. bis d. erzeugte Modell vollständig einbeschreibbar ist.

7. Vorrichtung zur Durchführung des Verfahrens nach einem der vorangehenden Ansprüche, **gekennzeichnet durch** eine Vorrichtung mit Baugruppen zur Durchführung der Verfahrensschritte durch Erzeugung von die Modelle repräsentierenden gespeicherten Daten mit Mitteln der Grafikdatenverarbeitung, beispielsweise mittels einer numerisch gesteuerten Werkzeugmaschine.

## Revendications

1. Procédé pour la fabrication d'une endoprothèse avec adaptation individuelle, en utilisant un modèle tridimensionnel généré par tomographie informatisée et existant au moins sous la forme de données, caractérisé en ce qu'il comprend les opérations:

a1. génération d'un modèle de comparaison pour les régions osseuses correspondantes de l'autre moitié – symétrique – du corps de la personne et inversion ou

a2. génération d'un modèle de comparaison pour les régions osseuses correspondantes d'après un modèle statistiquement moyen de la région osseuse concernée, adapté à la taille et aux autres particularités de la constitution de la personne, élaboré sur la base de valeurs statistiques moyennes, ainsi que

b. établissement des différences entre le modèle et le modèle de comparaison par leur comparaison dans une orientation spaciale adéquate,

c. détermination d'au moins une zone dans laquelle existe des écarts, par rapport au modèle de comparaison, qui dépassent une valeur préfixée en longueur ou en volume,

d. remplacement des zones divergentes par les zones correspondantes du modèle de comparaison, de même que

e. fabrication de l'endoprothèse selon le modèle ainsi obtenu.

2. Procédé selon la revendication 1, caractérisé par

a21. la génération du modèle de comparaison par interpolation entre les modèles statistiquement moyens, mémorisés.

3. Procédé selon la revendication 2, caractérisé par

a22. la génération du modèle de comparaison par variation de grandeur ou interpolation et comparaison du modèle statistiquement moyen avec les régions osseuses correspondantes de l'autre moitié du corps, avec inversion.

4. Procédé selon la revendication 1 ou 2, applicable surtout lorsque la région osseuse correspondante de la moitié opposée du corps n'est pas disponible à l'état non lésée, caractérisé par

a23. la génération du modèle de comparaison par variation de grandeur ou interpolation du modèle statistiquement moyen et comparaison avec les zones osseuses correspondantes de la moitité du corps à traiter, comprenant, selon un processus itératif, à la suite de l'opération d., une nouvelle adaptation selon l'opération a1., sans tenir compte des zons divergentes du modèle, selon c., et des zones remplacées du modèle, selon d.

5. Procédé selon l'une des revendications précédentes, caractérisé par

d1. l'adaptation exacte des sections de l'endoprothèse aux régions osseuses adjacentes.

6. Procédé selon l'une des revendications précédentes, caractérisé par

e1. la fabrication de l'endoprothèse à partir de l'ébauche la plus petite en volume de plusieurs ébauches, dans chacune desquelles peut être inscrit l'un de différents modèles générés selon un échelonnement préfixé et en fonction des différentes tailles des corps des individus, les portions de surface les plus éloignées d'un point central du modèle étant tout au plus tangentes aux surfaces extérieures brutes de l'ébauche dans laquelle peut être inscrit complètement le modèle généré selon les opérations a. jusqu'à d.

7. Dispositif pour la mise en œuvre du procédé selon l'une des revendications précédentes, caractérisé en ce qu'il comprend des unités pour exécuter les opérations du procédé par la génération de données, représentant les modèles et qui sont mémorisées, avec les moyens de la technique de traitement des données graphiques, la fabrication étant effectuée, par exemple, au moyen d'une machine-outil à commande numérique.

## Claims

1. A method of producing an endoprosthesis with individual adaptation, using a three-dimensional model produced by means of computer tomography and present at least in data-like representation, characterised by the steps:

a1. production of a reference model for the corresponding bone regions of the other – symmetrical – half of the person's body and mirror reversal, or

a2. production of a reference model for the corresponding bone regions from a statistical average model of the particular bone region, which model is adapted to the body size and the remaining constitution and based on statistical average values, and

b. discrimination between the surfaces of model and reference model by comparison in corresponding spatial alignment,

c. detecting at least one region in which there are deviations from the reference model which exceed a preset longitudinal or volumetric amount,

d. replacing the deviating regions by the corresponding regions of the reference model and

e. .production of the endoprosthesis from the model thus obtained.

2. A method according to. Claim 1, characterised by

a21. the production of the reference model by interpolation between the stored statistical average models.

3. A method according to Claim 2, characterised by

a22. the production of the reference model by dimensional variation or interpolation while matching the statistical average model with the corresponding bone regions of the other half of the body, with mirror reversal.

4. A method according to Claim 1 or 2, particularly if the corresponding bone region of the opposite half of the body is not available undamaged, characterised by

a23. the production of the reference model by dimensional variation or interpolation or the statistical average model while matching with the corresponding bone regions of the half of the body to be treated, wherein, after step d. a renewed adaptation is effected repeatedly in accordance with step a1. while ignoring the regions of the model differing according to c. and replaced according to d.

5. A method according to any one of the preceding Claims, characterised by

d1. the precise adaptation of the cross-sections of the endoprosthesis to the adjoining bone regions.

6. A method according to any one of the preceding Claims, characterised by

e1. the production of the endoprosthesis from the volumetrically smallest of a plurality of blanks which can each be inscribed in one of various models produced in a given graduation according to the different body sizes, while the parts of the surface furthest away from a central point of the model at most thouch the raw outer surfaces of the blank in which the model produced in accordance with steps a. to d. can be completely inscribed.

7. An apparatus for carrying out the method according to any one of the preceding claims, characterised by an apparatus with units for carrying out the method steps by producing stored data representing the models, with means for processing the graphic data, for example by means of a numerically controlled machine tool.